# EUROPÄISCHE PATENTSCHRIFT

(11) **EP 1 498 077 B1**
(45) Veröffentlichungstag und Bekanntmachung des Hinweises auf die Patenterteilung: **21.09.2005**
(21) Anmeldenummer: 03016022.0
(22) Anmeldetag: 15.07.2003
(51) Int. Cl.: A61B 17/28

(54) **Medizinisches Greif- und/oder Schneidinstrument**
Medical gripping and/or cutting instrument
Instrument medical de prise et/ou de coupe

(43) Veröffentlichungstag der Anmeldung: 19.01.2005
(73) Patentinhaber: University of Dundee, Dundee DD1 4HN (GB)
(72) Erfinder: Frank, Tim, Wormit, Fife (GB); Brown, Stuart I., St. Andrews KY16 8QX (GB); Rutherford, Ian, Dundee DD4 0RL (GB)
(74) Vertreter: Hofmeister, Frank

(56) Entgegenhaltungen:
- WO-A-03/101316
- US-A- 6 053 921
- US-A- 6 099 550

## Beschreibung

Die Erfindung betrifft ein medizinisches Greif- und/oder Schneidinstrument mit einem Instrumentenschaft, mit einen über einen Kopplungsmechanismus verlagerbar am Instrumentenschaft festlegbaren Handhabe sowie einem am distalen Ende des Instrumentenschaftes angeordneten, aus zwei Maulteilen bestehenden Werkzeug, wobei mindestens ein Maulteil des Werkzeugs relativ zu dem anderen Maulteil bewegbar ist.

Insbesondere bei Instrumenten für die laparoskopische Chirurgie weisen die medizinischen Instrumente häufig Schaftlängen von 30 cm und mehr auf. Diese Schaftlänge stellt sicher, dass auch weiter entfernt gelegene Operationsgebiete erreicht werden können, ohne den Instrumentenzugang verlegen zu müssen. Da die Instrumente jedoch die meiste Zeit für nahe gelegene Operationsgebiete verwendet werden, befindet sich der längste Teil des Instrumentenschaftes außerhalb des Körpers des Patienten. Daraus kann eine für den Operateur ungünstige und unbequeme Haltung bzw. Arbeitsstellung resultieren, da die Handhabe zum Führen und/oder Betätigen des chirurgischen Instruments am proximalen Ende des Instrumentenschafts angeordnet ist.

Dieses Problem wird bei der HALS Operationstechnik (Hand Assisted Laparoscopic Surgery) noch verstärkt, bei der zusätzlich zum Einbringen des Laparoskops und gegebenenfalls laparoskopischer Instrumente in die Bauchhöhle ein Hautschnitt zum Einführen einer Hand des Operateurs geschaffen wird, damit der Operateur per Tastsinn und unter Beobachtung und Kontrolle durch das Laparoskop eine besser geführte Operation durchführen kann. Während der Operateur mit einer Hand in der Bauchhöhle des Patienten die Operation unterstützt, betätigt er mit der anderen Hand die laparoskopischen Instrumente. Eine weit entfernt am Ende des Instrumentenschaftes angeordnete Handhabe erschwert die Arbeit des Operateurs dabei nicht unerheblich.

Zur Vermeidung der voranstehend beschriebenen Schwierigkeiten ist es bekannt, medizinische Instrumente mit einer verlagerbar am Instrumentenschaft festlegbaren Handhabe auszustatten.

Ein gattungsgemäßes medizinisches Instrument ist beispielsweise aus der US 6 053 921 A bekannt. Bei diesem bekannten medizinischen Instrument ist die Handhabe über einen federbelasteten Klemmmechanismus entlang der Längsachse des Instrumentenschaftes verlagerbar. Die Betätigung des distalseitig am Instrumentenschaft angeordneten Werkzeugs erfolgt über eine am proximalen Ende des Instrumentenschaftes angeordnete Betätigungseinheit. Diese räumliche Trennung der Handhabe einerseits und der Betätigungseinheit andererseits erschwert dem Operateur die Handhabung dieses Instruments, insbesondere unter räumlich erschwerten Arbeitsbedingungen.

Weiterhin ist es aus der Praxis bekannt, die Maulteile des Werkzeugs über einen oder mehrere, von der entlang der Instrumentenachse verstellbaren Handhabe ausgehende Bowdenzüge anzusteuern. Die Verwendung der Bowdenzüge hat jedoch den Nachteil, dass Bowdenzüge nur schlecht zu reinigen sind.

Davon ausgehend liegt der Erfindung die **Aufgabe** zugrunde, ein medizinisches Instrument der eingangs genannten Art zu schaffen, dass bei guter Reinigbarkeit und kompakter Bauweise eine einfache und sichere Handhabung gewährleistet.

Die **Lösung** dieser Aufgabenstellung ist erfindungsgemäß dadurch gekennzeichnet, dass der Kopplungsmechanismus zusätzlich zum Bedienen des mindestens einen bewegbaren Maulteils des Werkzeugs dient, und dass der Instrumentenschaft aus einem Innenschaft sowie einem den Innenschaft koaxial umgebenden Außenrohr, wobei das Außenrohr zumindest teilweise aus mehreren unabhängig voneinander in Längsrichtung des Instrumentenschaftes verschiebbaren Rohrabschnitten aufgebaut ist und die Handhabe auf der Außenseite des Außenrohres festlegbar ist.

Durch die erfindungsgemäße Ausgestaltung ist es möglich, sowohl das Festlegen der Handhabe am Instrumentenschaft als auch das Betätigen des Werkzeugs über nur ein Bauteil, nämlich den Kopplungsmechanismus, zu bewerkstelligen. Durch diese doppelte Funktion des Kopplungsmechanismus ist das Instrument sehr kompakt und somit für den Operateur einfach und sicher bedienbar. Insbesondere, wenn ein derartiges Instrument bei der HALS Operationstechnik Verwendung findet, ist diese kompakte Bauweise mit Doppelfunktion des Kopplungsmechanismus vorteilhaft.

Mit einer zweiten Ausführungsform der Erfindung wird vorgeschlagen, dass der Instrumentenschaft aus einem, einen Innenschaft bildenden, innenliegenden Zug-/Druckelement sowie einem das Zug-/Druckelement koaxial umgebenden Außenrohr besteht, wobei das Außenrohr zumindest proximalseitig eine Längsnut aufweist, über die der Kopplungsmechanismus mit dem Zug-/Druckelement in Eingriff bringbar ist. Die Betätigung des Zug-/Druckelements über den Kopplungsmechanismus erfolgt bei dieser Ausgestaltungsform dadurch, dass das Zug-/Druckelement zumindest abschnittsweise so ausgebildet ist, dass über die Handhabe ein zumindest axial festes Eingreifen auf das Zug-/Druckelement durch kraft- oder formschlüssige Verbindung gewährleistet ist, beispielsweise dadurch, dass das Zug-/Druckelement in diesem Bereich als Zahnstange ausgebildet ist. Ebenso ist es möglich, eine beispielsweise v-förmige Kerbe im Zug-/Druckelement vorzusehen, in die ein entsprechendes Eingriffselement der Handhabe einpressbar ist.

Vorteilhafterweise besteht die Handhabe aus dem aus zwei Klemmelementen aufgebauten Kopplungsmechanismus und einer zwischen den beiden Klemmelementen angeordneten Distanzhülse. Während die beiden Klemmelemente dazu dienen, zum einen die Handhabe am Instrumentenschaft zu fixieren und zum anderen die Maulteile des Werkzeugs zu betätigen, stellt die Distanzhülse den eigentlichen Teil dar, über den der Operateur die Handhabe zum Führen des Instruments ergreift. Zu diesem Zweck ist dieser zu ergreifende Teil vorzugsweise ergonomisch geformt ausgestaltet, oder aber an der Distanzhülse ein vorzugsweise ergonomisch geformt ausgestalteter Griffteil festlegbar.

Eine ortsfeste Fixierung der Handhabe am Außenrohr, bei der die Betätigung des Maulteils in allen axialen Positionen der Handhabe sichergestellt ist, ist vorteilhafterweise dadurch erzielbar, dass die axiale Erstreckung der Distanzhülse größer ist als die der einzelnen Rohrabschnitte des Außenrohres.

Zum Fixieren der Handhabe am Instrumentenschaft sowie zum Betätigen der Maulteile des Werkzeugs, die einerseits am distalen Ende des Innenschaftes und andererseits am distalen Ende des Außenrohres angeordnet sind, wird erfindungsgemäß vorgeschlagen, dass die vorteilhafterweise als Ringscheiben ausgebildeten und über ein Federelement in Richtung auf die Anlage an der Distanzhülse vorgespannten Klemmelemente einzeln und/oder gemeinsam gegenüber der Instrumentenlängsachse derart verkippbar sind, dass die Klemmelemente die Außenseite des Außenrohres bzw. die Außenseite von einem oder zwei Rohrabschnitten des Außenrohres klemmend ergreifen.

Das Verkippen der Klemmelemente gegenüber der Instrumentenlängsachse zum klemmenden Ergreifen des Außenrohres erfolgt erfindungsgemäß über mindestens eine an der Distanzhülse gelagerte Spreizvorrichtung, wobei vorteilhafterweise pro Klemmelement eine Spreizvorrichtung an der Distanzhülse vorgesehen ist, um die Klemmelemente auch unabhängig voneinander so verkippen zu können, dass die Handhabe durch das Verkippen nur eines Klemmelements am Außenrohr fixierbar ist.

Gemäß einer praktischen Ausführungsform der Erfindung wird vorgeschlagen, dass die Spreizvorrichtungen als mit Nocken versehene Kipphebel ausgebildet sind.

Zum Betätigen der am distalen Ende des Innenschaftes und am distalen Ende des Außenrohres angeordneten Maulteile des Werkzeugs sind die Rohrabschnitte des Außenrohres in der verkippten Stellung der Klemmelemente über die Klemmelemente in Längsrichtung des Instrumentenschaftes verschiebbar, wobei die axiale Erstreckung des Rohrabschnitts des Außenrohres, an dessen distalem Ende das eine Maulteil angeordnet ist, größer ist als die der einzelnen proximalseitigen Rohrabschnitte, vorzugsweise ein Mehrfaches der axialen Erstreckung der einzelnen proximalseitigen Rohrabschnitte beträgt.

Die Spreizvorrichtung zum Betätigen der Klemmelemente kann dabei so ausgebildet sein, dass beim Betätigen der Spreizvorrichtung zunächst die Klemmelemente nur zum klemmenden Ergreifen des Außenrohres verkippt werden underst ebim weiteren Betätigen der Spreizvorrichtung das Verschieben der Rohrabschnitte relativ zum Innenschaft erfolgt. Der Übergang vom Verkippen zum Verschieben kann dabei deutlich unterschiedliche Druckkräfte erkennbar ausgestaltet werden. Ebenso sind akkustische und/oder visuelle Begrenzungen möglich.

Um einerseits die proximalseitige Verschiebbarkeit des Außenrohres zu begrenzen und andererseits den Innenschaft über das verschiebbare Außenrohr in Richtung der Instrumentenlängsachse verlagern zu können, wird erfindungsgemäß vorgeschlagen, dass das Außenrohr kürzer ist als der Innenschaft und der Innenschaft proximalseitig einen Anschlag aufweist, der vorzugsweise als Anlagefläche für das proximale Ende des Außenrohres ausgebildet ist.

Weiterhin wird mit der Erfindung vorgeschlagen, dass zumindest die radial außen liegenden Umfangskanten der einzelnen Rohrabschnitte des Außenrohres abgeschrägt oder abgerundet ausgebildet sind, um die Handhabe verkantungsfrei entlang dem Außenrohr verschieben zu können. Das zusätzliche Abrunden der radial innen liegenden Umfangskanten gewährleistet ein störungsfreies Gleiten auf dem Innenschaft.

Um sicher zu stellen, dass die Maulteile des Werkzeugs nach der Betätigung über den Kopplungsmechanismus der Handhabe wieder in die Ausgangsstellung überführt werden, sind die Maulteile des Werkzeugs über ein Federelement in eine Arbeitsstellung vorgespannt. Ebenso wird mit der Erfindung vorgeschlagen, dass zumindest der aus den einzelnen Rohrabschnitten gebildete Teil des Außenrohrs über ein Federelement in proximale Richtung vorgespannt ist.

Schließlich wird mit der Erfindung vorgeschlagen, dass der jeweils proximalseitig über die Handhabe herausstehende Teil des Instrumentenschaftes gegenüber dem distalseitigen Teil des Instrumentenschaftes abwinkelbar ist, um die vom Operateur fortweisende proximale Länge des Instruments so kurz wie möglich auszugestalten.

Weitere Merkmale und Vorteile der Erfindung ergeben sich anhand der zugehörigen Zeichnung, in der ein Ausführungsbeispiel eines erfindungsgemäßen medizinischen Greif- und/oder Schneidinstruments nur beispielhaft dargestellt ist. In der Zeichnung zeigt:
- Fig. 1: eine schematische Seitenansicht eines erfindungsgemäßen medizinischen Greif- und/oder Schneidinstruments;
- Fig. 2: eine ausschnittweise perspektivische Draufsicht auf das Instrument gemäß Fig. 1;
- Fig. 3: eine um 90° gedrehte ausschnittweise Seitenansicht des Instruments gemäß Fig. 1, jedoch mit abgenommenem Griffteil;
- Fig. 4: eine vergrößerte ausschnittweise Seitenansicht gemäß Fig. 3, jedoch den Kopplungsmechanismus im einseitig betätigten Zustand darstellend;
- Fig. 5a: eine ausschnittweise schematische Darstellung der Arbeitsweise des erfindungsgemäßen Instruments, das Instrument in einer Ausgangsstellung darstellend und
- Fig. 5b: eine Fig. 5a entsprechende schematische Darstellung, jedoch das Instrument in einer Arbeitsstellung darstellend.

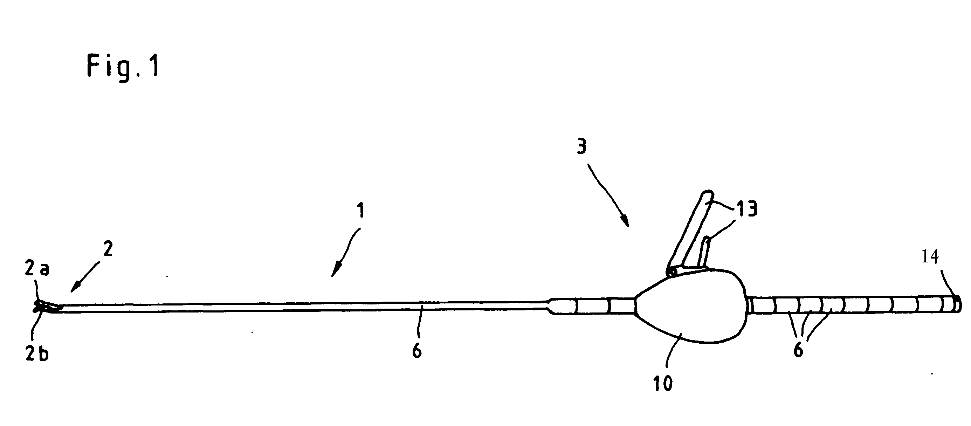

Das in Fig. 1 dargestellte medizinischen Greif- und/oder Schneidinstrument besteht im wesentlichen aus einem langgestreckten Instrumentenschaft 1, an dessen distalem Ende ein aus zwei Maulteilen 2a und 2b bestehendes Werkzeug 2 angeordnet ist, sowie einer Handhabe 3, die zumindest teilweise entlang dem Instrumentenschaft 1 verlagerbar am Instrumentenschaft 1 festlegbar ist.

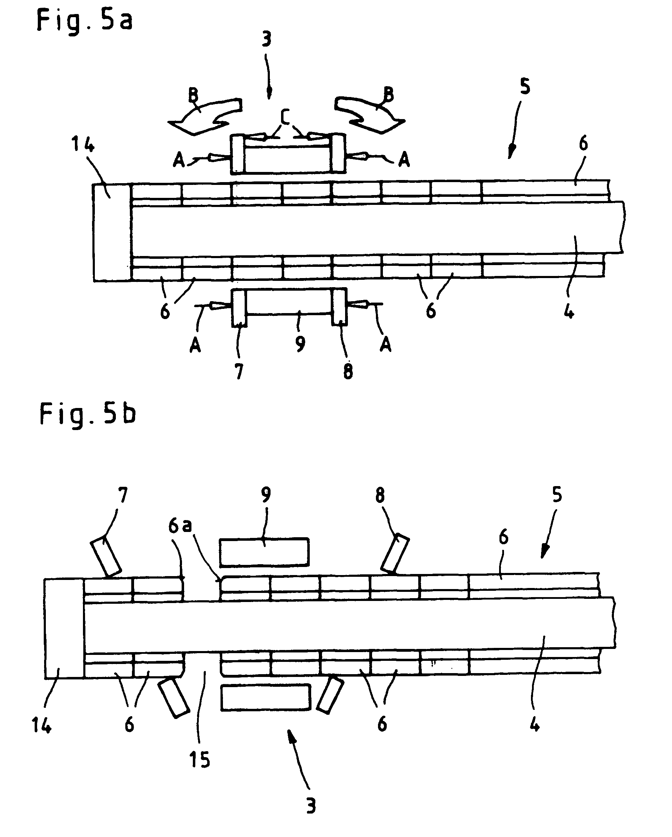

Wie aus Fig. 5a und 5b ersichtlich, besteht der Instrumentenschaft 1 aus einem Innenschaft 4 sowie einem den Innenschaft 4 koaxial umgebenden Außenrohr 5. Die am distalen Ende des Instrumentenschaftes 1 angeordneten Maulteile 2a und 2b des Werkzeugs 2 sind dabei so angeordnet, dass das Maulteil 2a am distalen Ende des Innenschaftes 4 gelagert ist und das Maulteil 2b am distalen Ende des Außenrohres 5 gelagert ist. Das Außenrohr 5 besteht bei dem dargestellten Ausführungsbeispiel aus mehreren unabhängig voneinander in Längsrichtung des Instrumentenschaftes 1 verschiebbaren Rohrabschnitten 6, wobei die axiale Erstrekkung des Rohrabschnitts 6 des Außenrohres 5, an dessen distalem Ende das eine Maulteil 2b angeordnet ist, ein Mehrfaches der axialen Erstreckung der einzelnen proximalseitigen Rohrabschnitte 6 beträgt. Zudem weist dieser Rohrabschnitt 6 einen gegenüber den anderen Rohrabschnitten 6 verringerten Außendurchmesser auf.

Auch, wenn die Rohrabschnitte 6 bei dem dargestellten Ausführungsbeispiel als im Querschnitt kreiszylindrische Rohrabschnitte 6 dargestellt sind, ist diese Querschnittsform nicht zwingend vorgeschrieben. Wichtig ist, dass die einzelnen Rohrabschnitte 6 in Längsrichtung des Instrumentenschaftes 1 relativ zum Innenschaft 4 verschiebbar sind. Vorzugsweise sind die Rohrabschnitte 6 auch verdrehsicher gegenüber dem Innenschaft 4, auf dem Innenschaft 4 gelagert. Dies ist beispielsweise dadurch erzielbar, dass die Rohrabschnitte Nuten oder Stege aufweisen und der Innenschaft 4 entsprechende Stege oder Nuten aufweist, die eine ausschließlich axiale Verlagerung der Rohrabschnitte 6 relativ zum Innenschaft 4 gewährleisten.

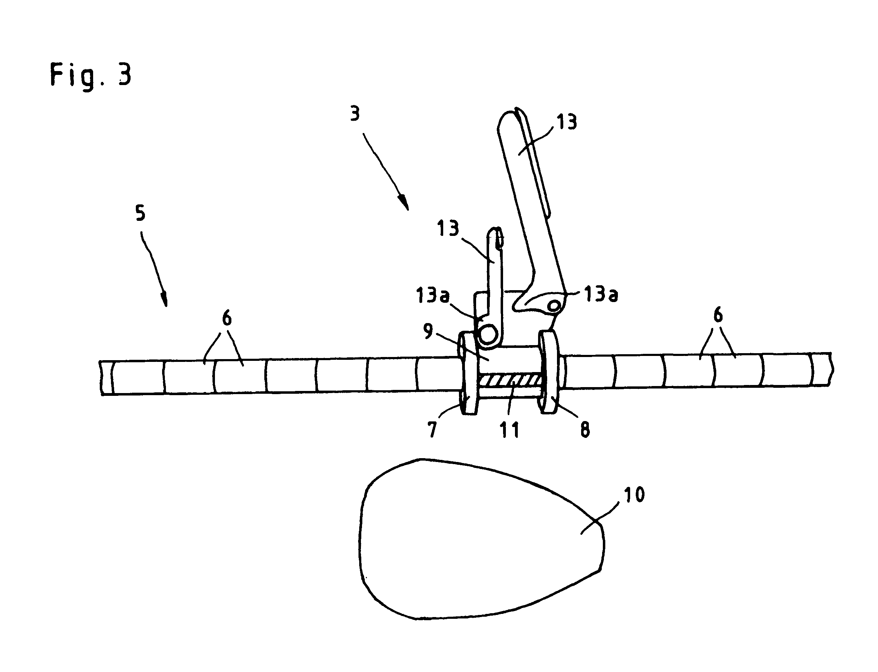

Die verlagerbar am Instrumentenschaft 1 festlegbare Handhabe 3 besteht, wie insbesondere aus Fig. 3 ersichtlich, aus einem aus zwei Klemmelementen 7, 8 gebildeten Kopplungsmechanismus und einer zwischen den beiden Klemmelementen 7, 8 angeordneten Distanzhülse 9 sowie einem an der Distanzhülse 9 festlegbaren Griffteil 10, wobei die axiale Erstreckung der Distanzhülse 9 größer ist als die der einzelnen proximalseitigen Rohrabschnitte 6 des Außenrohres 5. Der aus den beiden Klemmelementen 7 und 8 gebildete Kopplungsmechanismus dient einerseits zum Fixieren der Handhabe 3 am Instrumentenschaft 1 und andererseits zum Betätigen des Werkzeugs 2, wie dies nachfolgend noch näher beschrieben wird.

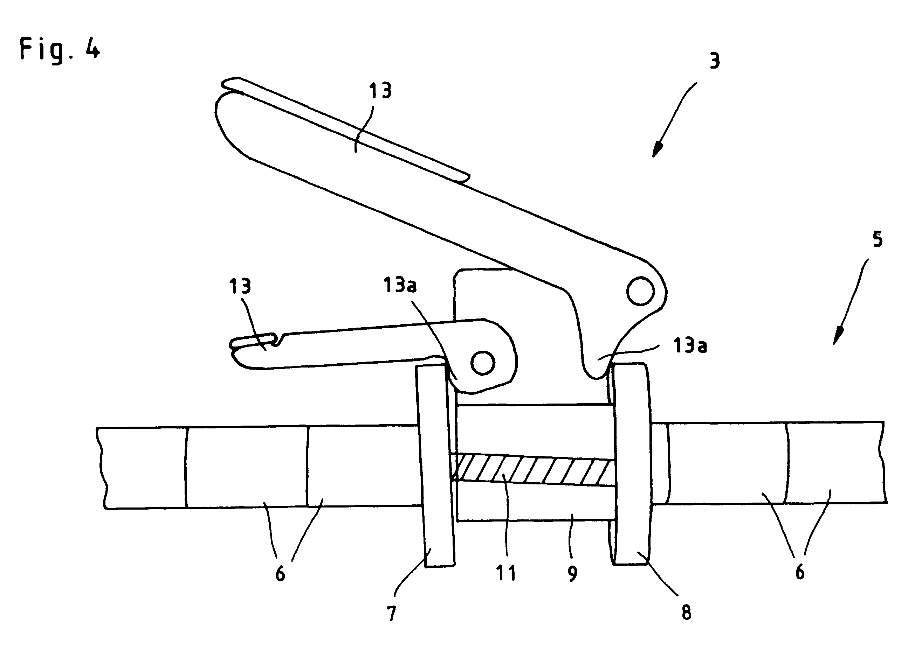

Wie aus Fig. 4 und 5b ersichtlich, sind die beiden über ein Federelement 11 in Richtung auf die Anlage an der Distanzhülse 9 vorgespannten Klemmelemente 7 und 8 des Kopplungsmechanismus der Handhabe 3 gegenüber der Instrumentenlängsachse 12 derart verkippbar, dass die Klemmelemente 7 und 8 die Außenseite des Außenrohres 5 klemmend bzw. verkantend ergreifen. Zum Verkippen der Klemmelemente 7 und 8 sind an der Distanzhülse 9 als Kipphebel 13 ausgebildete Spreizvorrichtungen gelagert, die über angeformte Nocken 13a die Klemmelemente 7, 8 einseitig derart mit einer Druckkraft beaufschlagen, dass die als Ringscheiben ausgebildeten Klemmelemente 7, 8 sich auf der Außenseite des Außenrohres 5 verkeilend/verkantend festlegen.

Bei der dargestellten Ausführungsform sind an der Distanzhülse zwei Kipphebel 13 gelagert, so dass pro Klemmelement 7, 8 jeweils ein Kipphebel 13 vorgesehen ist. Diese Ausgestaltung ermöglicht es, die Klemmelemente 7, 8 unabhängig voneinander über den jeweils zugehörigen Kipphebel 13 in die gegenüber der Instrumentenlängsachse 12 verkippte Stellung zu überführen. Selbstverständlich ist es auch möglich, beide Klemmelemente 7, 8 über eine gemeinsame Spreizvorrichtung zu verkippen.

Um die Handhabe 3 möglichst verkantungsfrei entlang der Außenseite des Außenrohres 5 verschieben zu können, sind die äußeren Umfangskanten 6a der Rohrabschnitte 6 abgeschrägt oder abgerundet ausgebildet, wie dies an einzelnen Rohrabschnitten gemäß Fig. 5b schematisch dargestellt ist. In ähnlicher Weise können die dem Innenschaft 4 zugewandten inneren Umfangskanten ebenfalls abgeschrägt oder abgerundet ausgestaltet sein, um auch ein verkantungsfreies verschieben der Rohrabschnitte 6 entlang dem Innenschaft 4 zu gewährleisten.

Die Arbeitsweise des dargestellten medizinischen Greif- und/oder Schneidinstruments wird nachfolgend insbesondere anhand der schematischen Zeichnungen Fig. 5a und Fig. 5b beschrieben:

Fig. 5a zeigt ausschnittweise schematisch den Aufbau des medizinischen Instruments mit dem Innenschaft 4 und dem den Innenschaft 4 koaxial umgebenden, aus einzelnen Rohrabschnitten 6 aufgebauten Außenrohr 5.

Die frei verschiebbar auf dem Innenschaft 4 gelagerten Rohrabschnitte 6 des Außenrohres 5 werden über ein nicht dargestelltes Federelement aneinander gedrückt und in die proximale Richtung so vorgespannt, dass die Rohrabschnitte 6 des Außenrohres 5 direkt an einem am proximalen Ende des Innenschaftes 4 angeordneten Anschlag 14 anliegen. Diese direkte Anlage der Rohrabschnitte 6 am Anschlag 14 des Innenschaftes 4 bewirkt, dass bei einem verschieben einzelner rohrabschnitte 6 zueinander unmittelbar auch eine Relativbewegung des Innenschaftes 4 zum Außenrohr 5 erzeugt wird, die wiederum unmittelbar eine Betätigung der an den distalen Enden des Innenschaftes 4 und des Außenrohres 5 angeordneten Maulteile 2a und 2b bewirkt.

In der in Fig. 5a dargestellten Ausgangsstellung ist die Handhabe 3 frei entlang dem Instrumentenschaft 1 verschiebbar, da über das Federelement 11 Zugkräfte in Richtung der Pfeile A auf die Klemmelemente 7 und 8 des Kopplungsmechanismus ausgeübt werden, die die Klemmelemente 7, 8 fest an die Distanzhülse 9 heranziehen.

Soll nun die Handhabe 3 am Instrumentenschaft 1 fixiert werden, werden einer oder beide, der aus Gründen der Übersichtlichkeit in diesen Schemazeichnungen nicht dargestellten Kipphebel 13 in Richtung der Pfeile B verschwenkt, wodurch die Klemmelemente 7, 8 über die Nocken 13a der Kipphebel 13 entgegen der Zugkraft des Federelements 11 aus der Anlage an der Distanzhülse 9 gedrückt und gegenüber der Instrumentenlängsachse 12 verkippt werden, wie dies der Abbildung Fig. 5b sowie dem linken Teil der Abbildung Fig. 4 zu entnehmen ist. Durch das Verkippen verkeilt sich das entsprechende Klemmelement 7, 8 auf der Außenseite des Außenrohres 5 und fixiert so die Handhabe 3 an den jeweiligen Rohrabschnitten 6 des Außenrohres 5.

Um sicherzustellen, dass die Rohrabschnitte 6 durch das Verkippen der Klemmelemente 7, 8 nicht deformiert oder anderweitig beschädigt werden, bestehen die einzelnen Rohrabschnitte 6 des Außenrohres 5 vorzugsweise aus einem sehr harten und inkompressiblen Material.

Da die axiale Erstreckung der Distanzhülse 9 größer ist als die der einzelnen Rohrabschnitte 6 des Außenrohres 5 verkeilen sich die Klemmelemente 7, 8 des Kopplungsmechanismus beim Verkippen auf verschiedenen der unabhängig voneinander verschiebbar auf dem Innenschaft 4 gelagerten Rohrabschnitte 6 des Außenrohres 5.

Werden nun die Kipphebel 13 weiter in Richtung der Pfeile B nach unten gedrückt, können die Klemmelemente 7, 8 nicht weiter verkippt werden, da sie bereits verklemmt auf der Außenseite der Rohrabschnitte 6 des Außenrohres festliegen. Das weitere Betätigen der Kipphebel 13 bewirkt somit ein Auseinanderdrücken der Klemmelemente 7, 8 in Richtung der Pfeile C gegen die Federkraft des Federelements 11. Da die einzelnen Klemmelemente 7 und 8 auf unterschiedlichen und voneinander unabhängigen Rohrabschnitten 6 des Außenrohres verkeilt fixiert sind, bewirkt diese Druckkraft in Richtung der Pfeile C ein Verschieben dieser jeweiligen Rohrabschnitte 6 in Richtung der Pfeile C, wodurch distalseitig ein Zwischenraum 15 zwischen einzelnen Rohrabschnitten 6 entsteht.

Wie aus Fig. 5b ersichtlich, bewirkt das Verschieben der Rohrabschnitte 6, die mit den Klemmelementen 7 und 8 in Eingriff stehen, sowie der Rohrabschnitte 6, die an diesen Rohrabschnitten 6 anliegen, distalseitig ein Verschieben des mit dem Maulteil 2b verbundenen Rohrabschnitts 6 des Außenrohres 5, wodurch das Maulteil 2b des Werkzeugs 2 betätigt, beispielsweise geschlossen, wird. Proximalseitig bewirkt das Verschieben der an dem Anschlag 14 des Innenschaftes 4 direkt anliegenden Rohrabschnitte 6 das der Innenschaft 4 aus Sicht der Darstellung gemäß Fig. 5b nach links gedrückt wird. Diese Verlagerung des Innenschaftes 4 bewirkt wiederum eine Betätigung des am distalen Ende des Innenschaftes 4 gelagerten Maulteils 2a.

Werden nun die Kipphebel 13 wieder in die Ausgangsstellung überführt, in der die Nocken 13a nicht mehr an den Klemmelementen 7, 8 angreifen, werden die Klemmelemente 7, 8 über das Federelement 11 wieder in die Anlage an der Distanzhülse 9 zurückgezogen, wodurch auch der Druck in Richtung der Pfeile C auf die Rohrabschnitte 6 aufgehoben wird. Das Überführen der Maulteile 2a, 2b des Werkzeugs 2 zurück in die beispielsweise geöffnete Ausgangsstellung erfolgt vorzugsweise über ein nicht dargestelltes Federelement. Ebenso werden die Rohrabschnitte 6 nach dem Lösen der Klemmelemente 7, 8 des Kopplungsmechanismus über das Federelement wieder in die in Fig. 5a dargestellte gegenseitige Anlage aneinander und am Anschlag 14 überführt. Das Vorspannen der Maulteile 2a, 2b sowie das Vorspannen der Rohrabschnitte 6 des Außenrohres 5 kann dabei über ein gemeinsames Federelement erfolgen.

Insgesamt zeichnet sich das dargestellte medizinische Instrument durch die Doppelfunktion des aus den Klemmelementen 7 und 8 gebildeten Kopplungsmechanismus der Handhabe 3 aus, der es einerseits ermöglicht, die Handhabe 3 an einer beliebigen Stelle des Instrumentenschaftes 1 zu fixieren und andererseits dazu dient, die Maulteile 2a, 2b des Werkzeugs 2 zu betätigen.

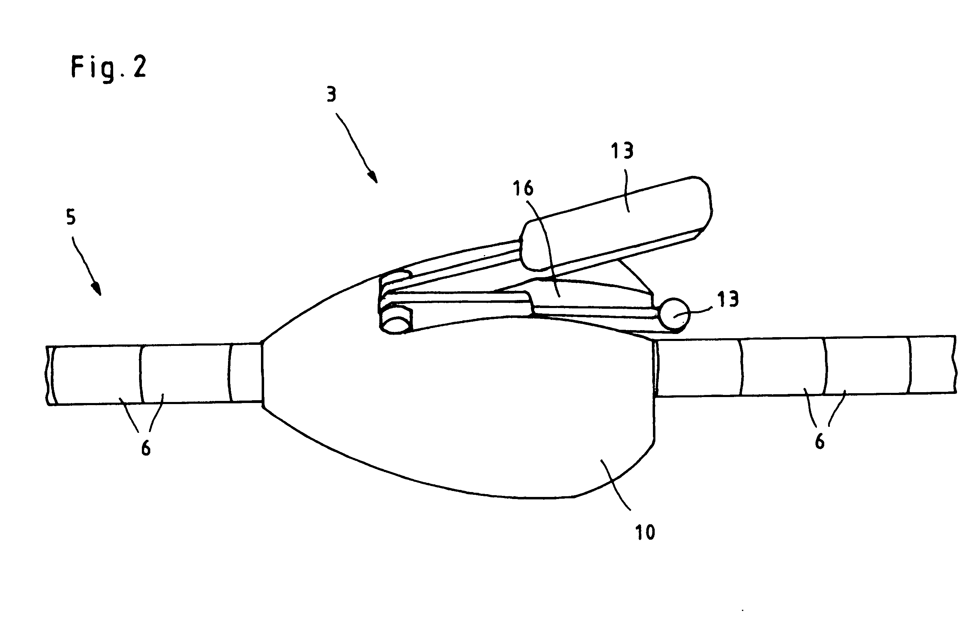

Wie insbesondere aus Fig. 1 und 2 ersichtlich, kann die Handhabbarkeit des Instruments dadurch vereinfacht werden, dass an der Distanzhülse 9 der Handhabe 3 ein ergonomisch geformtes Griffteil 10 festlegbar ist. Im vorliegenden Fall weist dieses Griffteil eine ovale "Eiform" auf, jedoch sind auch beliebige andere Formen denkbar, die in beiden Richtungen auf dem Instrumentenschaft festlegbar sind. Zum Betätigen der Kipphebel 13 weist das Griffteil 10 der dargestellten Ausführungsform eine Nut 16 auf, wie dies der Abbildung Fig. 2 zu entnehmen ist.

Alternativ zu der Verwendung des Griffteils 10 ist es selbstverständlich auch möglich, die Distanzhülse 9 selbst ergonomisch auszugestalten, so dass der Operateur das Instrument direkt über die Distanzhülse 9 führt.

### Bezugszeichenliste

- 1: Instrumentenschaft
- 2: Werkzeug
- 2a: Maulteil
- 2b: Maulteil
- 3: Handhabe
- 4: Innenschaft
- 5: Außenrohr
- 6: Rohrabschnitt
- 6a: Umfangskante
- 7: Klemmelement
- 8: Klemmelement
- 9: Distanzhülse
- 10: Griffteil
- 11: Federelement
- 12: Instrumentenlängsachse
- 13: Kipphebel
- 13a: Nocken
- 14: Anschlag
- 15: Zwischenraum
- 16: Nut

- A: Pfeil
- B: Pfeil
- C: Pfeil

## Patentansprüche

1. Medizinisches Greif- und/oder Schneidinstrument mit einem Instrumentenschaft (1), mit einer über einen Kopplungsmechanismus verlagerbar am Instrumentenschaft (1) festlegbaren Handhabe (3) sowie einem am distalen Ende des Instrumentenschaftes (1) angeordneten, aus zwei Maulteilen (2a, 2b) bestehenden Werkzeug (2), wobei mindestens ein Maulteil (2a oder 2b) des Werkzeugs (2) relativ zu dem anderen Maulteil (2b oder 2a) bewegbar ist,
**dadurch gekennzeichnet,**
**dass** der Kopplungsmechanismus zusätzlich zum Bedienen des mindestens einen bewegbaren Maulteils (2a oder 2b) des Werkzeugs (2) dient, und dass der Instrumentenschaft (1) aus einem Innenschaft (4) sowie einem den Innenschaft (4) koaxial umgebenden Außenrohr (5) besteht, wobei das Außenrohr (5) zumindest teilweise aus mehreren unabhängig voneinander in Längsrichtung des Instrumentenschaftes (1) verschiebbaren Rohrabschnitten (6) aufgebaut ist und die Handhabe (3) auf der Außenseite des Außenrohres (5) festlegbar ist.

2. Medizinisches Greif- und/oder Schneidinstrument nach Anspruch 1, **dadurch gekennzeichnet, dass** der Instrumentenschaft (1) aus einem, einen Innenschaft (4) bildenden, innenliegenden Zug-/Druckelement sowie einem das Zug-/Druckelement koaxial umgebenden Außenrohr (5) besteht, wobei das Außenrohr (5) zumindest proximalseitig eine Längsnut aufweist, über die der Kopplungsmechanismus mit dem Zug-/Druckelement in Eingriff bringbar ist.

3. Medizinisches Greif- und/oder Schneidinstrument nach Anspruch 2, **dadurch gekennzeichnet, dass** zur Betätigung des Zug-/Druckelements über den Kopplungsmechanismus das Zug-/Druckelement zumindest abschnittsweise als Zahnstange ausgebildet ist.

4. Medizinisches Greif- und/oder Schneidinstrument nach mindestens einem der Ansprüche 1 bis 3, **dadurch gekennzeichnet, dass** die Handhabe (3) aus dem aus zwei Klemmelementen (7, 8) aufgebauten Kopplungsmechanismus und einer zwischen den beiden Klemmelementen (7, 8) angeordneten Distanzhülse (9) besteht.

5. Medizinisches Greif- und/oder Schneidinstrument nach Anspruch 4, **dadurch gekennzeichnet, dass** die axiale Erstreckung der Distanzhülse (9) größer ist als die der einzelnen Rohrabschnitte (6) des Außenrohres (5).

6. Medizinisches Greif- und/oder Schneidinstrument nach Anspruch 4 oder 5, **dadurch gekennzeichnet, dass** die beiden Klemmelemente (7, 8) über ein Federelement (11) in Richtung auf die Anlage an der Distanzhülse (9) vorgespannt sind.

7. Medizinisches Greif- und/oder Schneidinstrument nach mindestens einem der Ansprüche 4 bis 6, **dadurch gekennzeichnet, dass** die Klemmelemente (7, 8) einzeln und/oder gemeinsam gegenüber der Instrumentenlängsachse (12) derart verkippbar sind, dass die Klemmelemente (7, 8) die Außenseite des Außenrohres (5) klemmend ergreifen.

8. Medizinisches Greif- und/oder Schneidinstrument nach mindestens einem der Ansprüche 4 bis 7, **dadurch gekennzeichnet, dass** die Klemmelemente (7, 8) als Ringscheiben ausgebildet sind.

9. Medizinisches Greif- und/oder Schneidinstrument nach Anspruch 7 oder 8, **dadurch gekennzeichnet, dass** die Klemmelemente (7, 8) über mindestens eine an der Distanzhülse (9) gelagerte Spreizvorrichtung gegenüber der Instrumentenlängsachse (12) verkippbar sind.

10. Medizinisches Greif- und/oder Schneidinstrument nach Anspruch 9, **dadurch gekennzeichnet, dass** pro Klemmelement (7, 8) eine Spreizvorrichtung an der Distanzhülse (9) vorgesehen ist.

11. Medizinisches Greif- und/oder Schneidinstrument nach Anspruch 9 oder 10, **dadurch gekennzeichnet, dass** die Spreizvorrichtungen als mit Nocken (13a) versehene Kipphebel (13) ausgebildet sind.

12. Medizinisches Greif- und/oder Schneidinstrument nach mindestens einem der Ansprüche 7 bis 11, **dadurch gekennzeichnet, dass** die Rohrabschnitte (6) des Außenrohres (5) in der verkippten Stellung der Klemmelemente (7, 8) über die Klemmelemente (7, 8) in Längsrichtung des Instrumentenschaftes (1) verschiebbar sind.

13. Medizinisches Greif- und/oder Schneidinstrument nach mindestens einem der Ansprüche 1 bis 12, **dadurch gekennzeichnet, dass** das Außenrohr (5) kürzer ist als der Innenschaft (4) und der Innenschaft (4) proximalseitig einen Anschlag (14) aufweist, der eine Anlagefläche für das proximale Ende des Außenrohres (5) bildet.

14. Medizinisches Greif- und/oder Schneidinstrument nach mindestens einem der Ansprüche 1 bis 13, **dadurch gekennzeichnet, dass** ein Maulteil (2a) des Werkzeugs (2) am distalen Ende des Innenschaftes (4) angeordnet ist und das andere Maulteil (2b) des Werkzeugs (2) am distalen Ende des Außenrohres (5) angeordnet ist.

15. Medizinisches Greif- und/oder Schneidinstrument nach Anspruch 14, **dadurch gekennzeichnet, dass** die axiale Erstreckung des Rohrabschnitts (6) des Außenrohres (5), an dessen distalem Ende das eine Maulteil (2b) angeordnet ist, ein Mehrfaches der axialen Erstreckung der einzelnen proximalseitigen Rohrabschnitte (6) beträgt.

16. Medizinisches Greif- und/oder Schneidinstrument nach Anspruch 1, **dadurch gekennzeichnet, dass** zumindest die radial außen liegenden Umfangskanten (6a) der einzelnen Rohrabschnitte (6) des Außenrohres (5) abgeschrägt oder abgerundet ausgebildet sind.

17. Medizinisches Greif- und/oder Schneidinstrument nach mindestens einem der Ansprüche 1 bis 16, **dadurch gekennzeichnet, dass** die Maulteile (2a, 2b) des Werkzeugs (2) über ein Federelement in eine Arbeitsstellung vorgespannt sind.

18. Medizinisches Greif- und/oder Schneidinstrument nach Anspruch 17, **dadurch gekennzeichnet, dass** zumindest der aus den einzelnen Rohrabschnitten (6) gebildete Teil des Außenrohrs (5) über ein Federelement in proximale Richtung vorgespannt ist.

19. Medizinisches Greif- und/oder Schneidinstrument nach mindestens einem der Ansprüche 4 bis 18, **dadurch gekennzeichnet, dass** die Distanzhülse (9) ergonomisch geformt ausgebildet ist.

20. Medizinisches Greif- und/oder Schneidinstrument nach mindestens einem der Ansprüche 4 bis 18, **dadurch gekennzeichnet, dass** an der Distanzhülse (9) ein vorzugsweise ergonomisch geformtes Griffteil (10) festlegbar ist.

21. Medizinisches Greif- und/oder Schneidinstrument nach mindestens einem der Ansprüche 1 bis 20, **dadurch gekennzeichnet, dass** der jeweils proximalseitig über die Handhabe (3) herausstehende Teil des Instrumentenschaftes (1) gegenüber dem distalseitigen Teil des Instrumentenschaftes (1) abwinkelbar ist.

## Claims

1. Medical gripping and/or cutting instrument with an instrument shaft (1), with a handle (3) which can be secured on the instrument shaft (1) and can be displaced via a coupling mechanism, and with a tool (2) which is arranged at the distal end of the instrument shaft (1) and comprises two jaw parts (2a, 2b), at least one jaw part (2a or 2b) of the tool (2) being movable relative to the other jaw part (2b or 2a), **characterized in that** the coupling mechanism additionally serves to operate the at least one movable jaw part (2a or 2b) of the tool (2), and **in that** the instrument shaft (1) is composed of an inner shaft (4) and of an outer tube (5) coaxially surrounding the inner shaft (4), the outer tube (5) being at least partly made up of several tube sections (6) which are displaceable independently of one another in the longitudinal direction of the instrument shaft (1), and the handle (3) being able to be secured on the outside of the outer tube (5).

2. Medical gripping and/or cutting instrument according to Claim 1, **characterized in that** the instrument shaft (1) is composed of an internal pull/push element forming an inner shaft (4) and of an outer tube (5) coaxially surrounding the pull/push element, said outer tube (5) having, at least at the proximal end, a longitudinal groove via which the coupling mechanism can be brought into engagement with the pull/push element.

3. Medical gripping and/or cutting instrument according to Claim 2, **characterized in that**, in order to actuate the pull/push element via the coupling mechanism, the pull/push element is formed at least in some sections as a toothed rack.

4. Medical gripping and/or cutting instrument according to at least one of Claims 1 to 3, **characterized in that** the handle (3) is composed of the coupling mechanism, made up of two clamp elements (7, 8), and of a spacer sleeve (9) arranged between the two clamp elements (7, 8).

5. Medical gripping and/or cutting instrument according to Claim 4, **characterized in that** the axial extent of the spacer sleeve (9) is greater than that of the individual tube sections (6) of the outer tube (5).

6. Medical gripping and/or cutting instrument according to Claim 4 or 5, **characterized in that** the two clamp elements (7, 8) are pretensioned via a spring element (11) in the direction of contact on the spacer sleeve (9).

7. Medical gripping and/or cutting instrument according to at least one of Claims 4 to 6, **characterized in that** the clamp elements (7, 8) can be tilted individually and/or jointly relative to the longitudinal axis (12) of the instrument in such a way that the clamp elements (7, 8) grip and clamp the outside of the outer tube (5).

8. Medical gripping and/or cutting instrument according to at least one of Claims 4 to 7, **characterized in that** the clamp elements (7, 8) are designed as annular discs.

9. Medical gripping and/or cutting instrument according to Claim 7 or 8, **characterized in that** the clamp elements (7, 8) can be tilted relative to the longitudinal axis (12) of the instrument via at least one spreading device mounted on the spacer sleeve (9).

10. Medical gripping and/or cutting instrument according to Claim 9, **characterized in that** one spreading device per clamp element (7, 8) is provided on the spacer sleeve (9).

11. Medical gripping and/or cutting instrument according to Claim 9 or 10, **characterized in that** the spreading devices are designed as tilt levers (13) provided with cams (13a).

12. Medical gripping and/or cutting instrument according to at least one of Claims 7 to 11, **characterized in that**, with the clamp elements (7, 8) in the tilted position, the tube sections (6) of the outer tube (5) can be moved in the longitudinal direction of the instrument shaft (1) via the clamp elements (7, 8).

13. Medical gripping and/or cutting instrument according to at least one of Claims 1 to 12, **characterized in that** the outer tube (5) is shorter than the inner shaft (4), and the inner shaft (4) has, at the proximal end, a stop (14) forming a contact surface for the proximal end of the outer tube (5).

14. Medical gripping and/or cutting instrument according to at least one of Claims 1 to 13, **characterized in that** one jaw part (2a) of the tool (2) is arranged at the distal end of the inner shaft (4), and the other jaw part (2b) of the tool (2) is arranged at the distal end of the outer tube (5).

15. Medical gripping and/or cutting instrument according to Claim 14, **characterized in that** the axial extent of the tube section (6) of the outer tube (5), at whose distal end the one jaw part (2b) is arranged, is a multiple of the axial extent of the individual tube sections (6) at the proximal end.

16. Medical gripping and/or cutting instrument according to Claim 1, **characterized in that** at least the radially outward peripheral edges (6a) of the individual tube sections (6) of the outer tube (5) are bevelled or rounded.

17. Medical gripping and/or cutting instrument according to at least one of Claims 1 to 16, **characterized in that** the jaw parts (2a, 2b) of the tool (2) are pretensioned into a working position via a spring element.

18. Medical gripping and/or cutting instrument according to Claim 17, **characterized in that** at least that part of the outer tube (5) formed by the individual tube sections (6) is pretensioned in the proximal direction via a spring element.

19. Medical gripping and/or cutting instrument according to at least one of Claims 4 to 18, **characterized in that** the spacer sleeve (9) has an ergonomic design.

20. Medical gripping and/or cutting instrument according to at least one of Claims 4 to 18, **characterized in that** a preferably ergonomically shaped grip part (10) can be secured on the spacer sleeve (9).

21. Medical gripping and/or cutting instrument according to at least one of Claims 1 to 20, **characterized in that** the part of the instrument shaft (1) protruding proximally from the handle (3) can be angled relative to the distal part of the instrument shaft (1).

## Revendications

1. Instrument médical de prise et/ou de coupe avec une tige d'instrument (1), avec un dispositif de maniement (3) qui peut être fixé sur la tige d'instrument (1) de manière déplaçable au moyen d'un mécanisme d'accouplement, ainsi qu'un outil (2) constitué de deux parties de mâchoire (2a, 2b) placé à l'extrémité distale de la tige d'instrument (1), au moins une partie de mâchoire (2a ou 2b) de l'outil (2) étant déplaçable par rapport à l'autre partie de mâchoire (2b ou 2a),
**caractérisé en ce que**
le mécanisme d'accouplement sert, en plus, à actionner la au moins une partie de mâchoire déplaçable (2a ou 2b) de l'outil (2) et **en ce que** la tige d'instrument (1) est constituée d'une tige intérieure (4) ainsi que d'un tube extérieur (5) entourant coaxialement la tige intérieure (4), le tube extérieur (5) étant formé, au moins partiellement, par plusieurs tronçons de tube (6) déplaçables indépendamment les uns des autres dans la direction longitudinale de la tige d'instrument (1) et le dispositif de maniement (3) pouvant être fixé sur le côté extérieur du tube extérieur (5).

2. Instrument médical de prise et/ou de coupe selon la revendication 1, **caractérisé en ce que** la tige d'instrument (1) est constituée d'un élément de traction/pression placé à l'intérieur formant une tige intérieure (4) ainsi que d'un tube extérieur (5) entourant coaxialement l'élément de traction/pression, le tube extérieur (5) comportant, au moins du côté proximal, une rainure longitudinale par l'intermédiaire de laquelle le mécanisme d'accouplement peut être mis en prise avec l'élément de traction/pression.

3. Instrument médical de prise et/ou de coupe selon la revendication 2, **caractérisé en ce que**, pour actionner l'élément de traction/pression par l'intermédiaire du mécanisme d'accouplement, l'élément de traction/pression est configuré comme une crémaillère, au moins par tronçons.

4. Instrument médical de prise et/ou de coupe selon au moins l'une quelconque des revendications 1 à 3, **caractérisé en ce que** le dispositif de maniement (3) est constitué du mécanisme d'accouplement formé de deux éléments de serrage (7, 8) et d'une douille d'écartement (9) placée entre les deux éléments de serrage (7, 8).

5. Instrument médical de prise et/ou de coupe selon la revendication 4, **caractérisé en ce que** l'allongement axial de la douille d'écartement (9) est supérieur à celui des tronçons de tube individuels (6) du tube extérieur (5).

6. Instrument médical de prise et/ou de coupe selon la revendication 4 ou 5, **caractérisé en ce que** les deux éléments de serrage (7, 8) sont précontraints par un élément de ressort (11) sur la douille d'écartement (9) dans la direction du contact.

7. Instrument médical de prise et/ou de coupe selon au moins l'une quelconque des revendications 4 à 6, **caractérisé en ce que** les éléments de serrage (7, 8) sont renversables individuellement et/ou conjointement par rapport à l'axe longitudinal de l'instrument (12) de manière à ce que les éléments de serrage (7, 8) saisissent le côté extérieur du tube extérieur (5) en le serrant.

8. Instrument médical de prise et/ou de coupe selon au moins l'une quelconque des revendications 4 à 7, **caractérisé en ce que** les éléments de serrage (7, 8) sont conçus comme des disques annulaires.

9. Instrument médical de prise et/ou de coupe selon la revendication 7 ou 8, **caractérisé en ce que** les éléments de serrage (7, 8) sont renversables par rapport à l'axe longitudinal de l'instrument (12) au moyen d'au moins un dispositif écarteur logé sur la douille d'écartement (9).

10. Instrument médical de prise et/ou de coupe selon la revendication 9, **caractérisé en ce qu'**un dispositif écarteur est prévu sur la douille d'écartement (9) pour chaque élément de serrage (7, 8).

11. Instrument médical de prise et/ou de coupe selon la revendication 9 ou 10, **caractérisé en ce que** les dispositifs écarteurs sont conçus comme des leviers oscillants (13) munis d'ergots (13a).

12. Instrument médical de prise et/ou de coupe selon au moins l'une quelconque des revendications 7 à 11, **caractérisé en ce que** les tronçons de tube (6) du tube extérieur (5) sont déplaçables dans la direction longitudinale de la tige d'instrument (1) au moyen des éléments de serrage (7, 8) dans la position renversée des éléments de serrage (7, 8).

13. Instrument médical de prise et/ou de coupe selon au moins l'une quelconque des revendications 1 à 12, **caractérisé en ce que** le tube extérieur (5) est plus court que la tige intérieure (4) et **en ce que** la tige intérieure (4) comporte une butée (14) du côté proximal qui forme une surface de contact pour l'extrémité proximale du tube extérieur (5).

14. Instrument médical de prise et/ou de coupe selon au moins l'une quelconque des revendications 1 à 13, **caractérisé en ce qu'**une partie de mâchoire (2a) de l'outil (2) est placée à l'extrémité distale de la tige intérieure (4) et l'autre partie de mâchoire (2b) de l'outil (2) est placée à l'extrémité distale de du tube extérieur (5).

15. Instrument médical de prise et/ou de coupe selon la revendication 14, **caractérisé en ce que** l'allongement axial du tronçon de tube (6) du tube extérieur (5) à l'extrémité distale duquel est placée une partie de mâchoire (2b) est un multiple de l'allongement axial des tronçons de tube individuels (6) du côté proximal.

16. Instrument médical de prise et/ou de coupe selon la revendication 1, **caractérisé en ce qu'**au moins les arêtes circonférentielles (6a) des tronçons de tube individuels (6) du tube extérieur (5) placées à l'extérieur dans le sens radial sont biseautées ou arrondies.

17. Instrument médical de prise et/ou de coupe selon au moins l'une quelconque des revendications 1 à 16, **caractérisé en ce que** les parties de mâchoire (2a, 2b) de l'outil (2) sont précontraintes par un élément de ressort dans une position de travail.

18. Instrument médical de prise et/ou de coupe selon la revendication 17, **caractérisé en ce qu'**au moins la partie du tube extérieur (5) formée par les tronçons de tube individuels (6) est précontrainte par un élément de ressort dans la direction proximale.

19. Instrument médical de prise et/ou de coupe selon au moins l'une quelconque des revendications 4 à 18, **caractérisé en ce que** la douille d'écartement (9) est conçue selon une forme ergonomique.

20. Instrument médical de prise et/ou de coupe selon au moins l'une quelconque des revendications 4 à 18, **caractérisé en ce qu'**une poignée (10) ayant, de préférence, une forme ergonomique peut être fixée sur la douille d'écartement (9).

21. Instrument médical de prise et/ou de coupe selon au moins l'une quelconque des revendications 1 à 20, **caractérisé en ce que** la partie de la tige d'instrument (1) faisant saillie au dessus du dispositif de maniement (3) du côté proximal peut être pliée en U par rapport à la partie de la tige d'instrument (1) du côté distal.
